# EUROPEAN PATENT SPECIFICATION

(11) **EP 2 550 954 B1**
(45) Date of publication and mention of the grant of the patent: **04.03.2020**
(21) Application number: 11759303.8
(22) Date of filing: 17.03.2011
(51) Int. Cl.: A61K 8/37, A61K 8/31, A61K 8/63, A61K 8/81, A61K 8/85, A61Q 1/02, A61Q 1/04, A61Q 1/12, A61Q 19/00

(54) **VASELINE-LIKE COMPOSITION, AND COSMETIC PREPARATION**
VASELINEARTIGE ZUSAMMENSETZUNG UND KOSMETIKPRÄPARAT DARAUS
COMPOSITION DE TYPE VASELINE, ET PRÉPARATION COSMÉTIQUE

(30) Priority: 25.03.2010 JP 2010070136
(43) Date of publication of application: 30.01.2013
(73) Proprietor: The Nisshin OilliO Group, Ltd., Tokyo 104-8285 (JP)
(72) Inventor: KACHI Hisanori, Yokohama-shi Kanagawa 235-8558 (JP); MATSUZAWA Makoto, Yokohama-shi Kanagawa 235-8558 (JP)
(74) Representative: Cabinet Laurent & Charras
(86) International application number: PCT/JP2011/056380
(87) International publication number: WO 2011/118497

(56) References cited:
- WO-A1-2006/080389
- JP-A- 2000 204 032
- JP-A- 2001 158 718
- JP-A- 2003 226 609
- JP-A- 2003 286 128
- JP-A- 2007 269 746
- US-A1- 2006 204 460
- US-A1- 2007 269 470
- US-A1- 2008 260 663
- ANONYMOUS: "Kobo Microspheres & Microsphere Complexes", INTERNET CITATION, May 2003 (2003-05), pages 1-16, XP002541909, Retrieved from the Internet: URL:http://web.archive.org/web/20060212052 550/www.koboproductsinc.com/Downloads/Micr ospheres-MMC003b.pdf [retrieved on 2009-08-17]

## Description

### TECHNICAL FIELD

The present invention relates to a vaseline-like composition having moisture occlusion comparable to that of vaseline, superior spreadability on skin, little stickiness and favorable skin blendability, and to a cosmetic incorporating this composition.

The present application claims priority on the basis of Japanese Patent Application No. 2010-070136, filed in Japan on March 25, 2010, the contents of which are incorporated herein by reference.

### BACKGROUND ART

Vaseline is obtained by purifying an ointment-like substance obtained by solvent dewaxing of a vacuum-distilled residual oil of crude oil, and is mainly composed of amorphous hydrocarbons having 24 to 34 carbon atoms. Highly purified white vaseline capable of being used in cosmetics and pharmaceuticals is available commercially.

Vaseline is frequently used in skin care cosmetics, makeup cosmetics and pharmaceuticals externally applied to the skin. Its most important functions consist of a moisture retention function and moisture transpiration inhibitory function (to be referred to as "moisture occlusion") for imparting moisture and softness to skin.

Skin problems such as rough skin are frequently caused by drying of the skin, and as a remedy for this, the application of vaseline and the like to effectively inhibit moisture transpiration from the skin is known to improve these skin problems. Consequently, products incorporating vaseline are available commercially in the form of cosmetics or external preparations demonstrating high levels of moisture occlusion.

Moisture occlusion produced by oily agents is known to be dependent on the polarity of the oily agent, and vaseline having low polarity demonstrates a high level of moisture occlusion, while ester oils having high polarity demonstrate a low level of moisture occlusion.

However, although vaseline is soft and has favorable spreadability, since it typically has difficulty in blending with the skin, after having applied a cream and the like, which contains non-polar oils including vaseline as the main components thereof, over vaseline after having applied vaseline to the skin, there are a significant number of times in which this creates problems during the course of daily life, such as the hand used to apply the cream remaining sticky for a long period of time, soiling of clothing and the like.

On the other hand, diisostearyl malate and diglyceryl triisostearate are frequently used in makeup cosmetics as oily agents having suitable viscosity and superior pigment dispersibility. However, since these substances have superior air permeability, they are far inferior to vaseline in terms of moisture occlusion, and have little ameliorative effect on rough skin.

In addition, since hydrocarbon-based waxes such as polyethylene wax, paraffin wax, ceresin wax or ozocerite form a solid that entraps liquid oils in wax when cooled after having been heated and mixed with a liquid oil, these substances are frequently used in oily solid cosmetics such as lipstick. However, since these waxes have a high degree of crystallinity, they inevitably produce a hard sensation when applied to skin, thereby making it difficult to realize smooth spreadability in the manner of vaseline. In the case of reducing the incorporated amount of wax in order to prevent this, the liquid oil ends up seeping out over time, thereby resulting in the problem of a decrease in product value.

Patent Document 1 discloses a water-in-oil type emulsified external skin preparation having favorable moisture occlusion that incorporates ester oil, a specific ester compound and a water-soluble inorganic salt. However, the specific ester compound indicated in the examples (glyceryl (behenate/ eicosadioate)) is not in the form of a water-in-oil emulsion, and although it demonstrates high moisture occlusion in the oil phase only, since it is a solid composition, the skin spreadability and storage stability of an external skin preparation incorporating this ester compound were not satisfactory.

With the foregoing in view, there has been a desire for a vaseline-like composition that realizes moisture occlusion comparable to that of vaseline, has superior skin spreadability, superior skin blendability and little stickiness.

### [Prior Art Documents]

### [Patent Documents]

[Patent Document 1] Japanese Unexamined Patent Application, First Publication No. 2003-212747

### DISCLOSURE OF THE INVENTION

### [Problems to be Solved by the Invention]

An object of the present invention is to provide a vaseline-like composition that realizes moisture occlusion comparable to that of vaseline, has superior skin spreadability, superior skin blendability and little stickiness, and a cosmetic that incorporates this composition.

### [Means for Solving the Problems]

As a result of conducting extensive studies to solve the aforementioned problems, the inventors of the present invention found that by mixing: I) one or more types of ester compounds selected from diisostearyl malate and diglyceryl triisostearate, II) a hydrocarbon wax having a melting point of 70°C to 130°C, and III) at least one type of compound selected from poly(12-hydroxystearic acid) having an average degree of polymerization of 3 to 20, a poly(12-hydroxystearic acid) derivative obtained by esterifying poly(12-hydroxystearic acid) having an average degree of polymerization of 3 to 20 and a polyvalent alcohol having valence of 5 or more, and a dimer dilinoleic acid derivative at a specific ratio, a composition is obtained that realizes moisture occlusion comparable to that of vaseline, superior skin spreadability, superior skin blendability and little stickiness, thereby leading to completion of the present invention.

### [Effects of the Invention]

According to the present invention, a vaseline-like composition that realizes moisture occlusion comparable to that of vaseline, has superior skin spreadability, superior skin blendability and little stickiness and can serve as a raw material of various types of cosmetics, and a cosmetic incorporating the vaseline-like composition, can be provided.

### BEST MODE FOR CARRYING OUT THE INVENTION

The following provides a detailed explanation of the present invention. The vaseline-like composition of the present invention consisting of the following components (C) or (E) at 5% by weight to 20% by weight, the following component (A) at 40% by weight to 90% by weight, and the following component (B) at 5% by weight to 50% by weight:
(A) one or more types of ester compounds selected from diisostearyl malate and diglyceryl triisostearate;
(B) a hydrocarbon wax having a melting point of 70°C to 130°C;
(C) a poly(12-hydroxystearic acid) having an average degree of polymerization of 3 to 20;
   and,
(E) a dimer dilinoleic acid derivative.

The vaseline-like composition of the present invention contains one or more types of ester compounds selected from diisostearyl malate and diglyceryl triisostearate as component (A) .

The incorporated amount of component (A) is 40% by weight to 90% by weight, preferably 50% by weight to 90% by weight, more preferably 60% by weight to 90% by weight, even more preferably 70% by weight to 90% by weight, and particularly preferably 80% by weight to 90% by weight based on the weight of the vaseline-like composition. As a result of making the incorporated amount of component (A) to be within these ranges, a vaseline-like composition can be prepared that has superior moisture occlusion, superior skin spreadability and blendability, and little stickiness.

The vaseline-like composition of the present invention contains a hydrocarbon wax having a melting point of 70°C to 130°C as component (B). As a result of making the melting point to be 70°C to 130°C, a vaseline-like composition can be prepared that has superior moisture occlusion, superior skin spreadability and blendability, and little stickiness.

The hydrocarbon wax having a melting point of 70°C to 130°C is preferably one or more types selected from polyethylene wax, paraffin wax, ceresin wax and ozocerite. One type of the aforementioned hydrocarbon wax may be used alone or two or more types may be used in combination.

The incorporated amount of component (B) is 5% by weight to 50% by weight, preferably 5% by weight to 40% by weight, more preferably 5% by weight to 30% by weight, and even more preferably 5% by weight to 20% by weight based on the weight of the vaseline-like composition. If the incorporated amount of component (B) is made to be within these ranges, a vaseline-like composition can be prepared that has superior moisture occlusion, superior skin spreadability and blendability, and little stickiness.

The vaseline-like composition of the present invention contains one type or two or more types of components selected from components (C) or (E).

In the present invention, component (C) is a poly(12-hydroxystearic acid) having an average degree of polymerization of 3 to 20. As a result of their incorporation, a vaseline-like composition can be prepared that has superior moisture occlusion, superior skin spreadability and blendability, and little stickiness.

The poly (12-hydroxystearic acid) of the present invention is a polymer of 12-hydroxystearic acid having a single hydroxyl group in a molecule thereof. Since 12-hydroxystearic acid can be obtained by, for example, hydrogenating ricinoleic acid obtained by hydrolyzing castor seed oil, poly(12-hydroxystearic acid) can be obtained by carrying out a polymerization reaction of the aforementioned 12-hydroxystearic acid. The polymerization reaction of 12-hydroxystearic acid is an intermolecular esterification reaction consisting of esterification of a hydroxyl group or carboxyl group in a molecule of 12-hydroxystearic acid with a hydroxyl group or carboxylic acid group in another molecule of 12-hydroxystearic acid. There are no particular limitations on the esterification method, and esterification can be carried out in accordance with ordinary methods.

The average degree of polymerization of the poly(12-hydroxystearic acid) is 3 to 20, preferably 5 to 20, more preferably 5 to 15 and particularly preferably 5 to 10. If the average degree of polymerization is within the aforementioned ranges, a vaseline-like composition can be prepared that has superior moisture occlusion, superior skin spreadability and blendability, and little stickiness. The average degree of polymerization can be made to be within the aforementioned ranges by measuring the acid value of the reaction product during the course of the polymerization reaction of 12-hydroxystearic acid. Namely, this can be easily carried out by sampling the reaction product during the course of the polymerization reaction of 12-hydroxystearic acid, calculating the average degree of polymerization by measuring the acid value thereof, and terminating the esterification reaction at the point the average degree of polymerization has reached a desired average degree of polymerization.

In the present invention, component (E) is a dimer dilinoleic acid derivative. As a result of incorporating a dimer dilinoleic acid derivative, a vaseline-like composition can be prepared that has superior moisture occlusion, superior skin spreadability and blendability, and little stickiness.

In the present invention the dimer dilinoleic acid derivative, is an ester of an alcohol and one or more types of acids selected from dimer dilinoleic acid and hydrogenated dimer dilinoleic acid.

The dimer dilinoleic acid refers to a known dibasic acid obtained by dimerizing an unsaturated fatty acid composed mainly of linoleic acid by an intermolecular polymerization reaction.

A hydrogenated dimer dilinoleic acid can be obtained by reducing (hydrogenating) the aforementioned dimer dilinoleic acid.

The alcohol which forms an ester bond with one or more types of acids selected from dimer dilinoleic acid and hydrogenated dimer dilinoleic acid, is one or more types of alcohols selected from cholesterol, phytosterol and monovalent alcohols having 4 to 22 carbon atoms. In addition, the monovalent alcohols having 4 to 22 carbon atoms may be saturated or unsaturated and linear or branched.

More specifically, one or more types selected from cholesterol, phytosterol and monovalent alcohols having 4 to 22 carbon atoms are preferably used for the ester-bonding alcohol. The essential use of one or more types of alcohols selected from cholesterol and phytosterol is more preferable. The use of one or more types of alcohols selected from cholesterol and phytosterol and one or more types of alcohols selected from an saturated or unsaturated, linear or branched monovalent alcohol having 4 to 22 carbon atoms is even more preferable. The use of one or more types of alcohols selected from cholesterol and phytosterol and one or more types of alcohols selected from stearyl alcohol, isostearyl alcohol cetyl alcohol is most preferable.

When the total amount of alcohol that ester-bonds per 1 molecule of dimer dilinoleic acid or hydrogenated dimer dilinoleic acid is assigned a molar ratio of 1, the content of cholesterol and phytosterol is preferably a molar ratio of 0.05 to 0.9, more preferably a molar ratio of 0.1 to 0.8, and even more preferably a molar ratio of 0.2 to 0.7.

One type of the aforementioned component (E) may be used alone or two or more types may be used in combination.

In the present invention, only one type of components (C) or (E) may be used, or two or more types may be used in combination. In the case of using only one type of components (C)or (E), superior effects are obtained by using component (E).

The total incorporated amount of components (C) and (E) based on the weight of the vaseline-like composition is 5% by weight to 20% by weight, preferably 5% by weight to 15% by weight, and more preferably 5% by weight to 12% by weight. As a result of incorporating these components within these ranges, a vaseline-like composition can be prepared that has superior moisture occlusion, superior skin spreadability and blendability, and little stickiness.

Various types of components typically used in compositions for use in vaseline-like compositions and cosmetic applications can be incorporated in the vaseline-like composition of the present invention in addition to the aforementioned components within a range that does not impair the effects of the present invention. More specifically, water, oily components other than components (A) to (C), anionic surfactants, cationic surfactants, amphoteric surfactants, nonionic surfactants, powdered components, moisturizers, natural water-soluble polymers, semi-synthetic water-soluble polymers, synthetic water-soluble polymers, inorganic water-soluble polymers, ultraviolet absorbers, metal ion sequestering agents, lower alcohols, polyvalent alcohols, monosaccharides, oligosaccharides, amino acids, organic amines, synthetic resin emulsions, pH regulators, vitamins, antioxidants, antioxidant synergists, antiseptics, salts or fragrances and the like can be suitably incorporated as necessary.

Water typically used in cosmetics is used for the water, and water such as ion exchange water, distilled water, fruit or vegetable-derived water or desalinated seawater can be used without any particular limitations thereon.

Examples of oily components other than the oily components serving as essential components of the present invention include hydrocarbon oils, synthetic ester oils, animal and vegetable oils and fats, silicone oils, higher fatty acids, higher alcohols and phospholipids, and one or more types of any of these oily components can be used. Oily components that are a liquid, semi-solid or solid at normal temperatures can be used, and there are no particular limitations thereon.

Examples of hydrocarbon oils include light liquid isoparaffin, liquid paraffin, polydecene, hydrogenated polydecene, polyisobutene, hydrogenated polyisobutene, squalane, squalene, α-olefin oligomers and pristane.

Examples of synthetic ester oils include 2-pentylnonyl hexanoate, 2-hexyldecyl hexanoate, 2-heptylundecyl hexanoate, 2-octyldecyl hexanoate, isostearyl hexanoate, 2-butyloctyl octanoate, isotridecyl octanoate, 2-pentylnonyl octanoate, 2-hexyldecyl octanoate, 2-heptylundecyl octanoate, 2-octyldodecyl octanoate, isostearyl octanoate, 2-propylheptyl decanoate, 2-butyloctyl decanoate, isotridecyl decanoate, 2-pentylnonyl decanoate, 2-hexyldecyl decanoate, 2-heptylundecyl decanoate, 2-octyldodecyl decanoate, isostearyl decanoate, 2-propylheptyl laurate, isononyl laurate, 2-butyloctyl laurate, isotridecyl laurate, 2-pentylnonyl laurate, 2-hexyldecyl laurate, 2-heptylundecyl laurate, 2-octyldodecyl laurate, isotridecyl laurate, isostearyl laurate, 2-ethylhexyl myristate, isononyl myristate, 2-propylheptyl myristate, 2-butyloctyl myristate, isotridecyl myristate, 2-pentylnonyl myristate, 2-hexyldecyl myristate, 2-heptylundecyl myristate, 2-octyldodecyl myristate, isostearyl myristate, isobutyl palmitate, 2-ethylhexyl palmitate, isononyl palmitate, 2-propylheptyl palmitate, 2-butyloctyl palmitate, isotridecyl palmitate, 2-pentylnonyl palmitate, 2-hexyldecyl palmitate, 2-heptylundecyl palmitate, 2-octyldodecyl palmitate, isostearyl palmitate, isopropyl stearate, isobutyl stearate, 2-ethylhexyl stearate, isononyl stearate, 2-propylheptyl stearate, 2-butyloctyl stearate, isotridecyl stearate, 2-pentylnonyl stearate, 2-hexyldecyl stearate, 2-heptylundecyl stearate, 2-octyldodecyl stearate, isostearyl stearate, isopropyl behenate, isobutyl behenate, 2-ethylhexyl behenate, isononyl behenate, 2-propylheptyl behenate, 2-butyloctyl behenate, isotridecyl behenate, 2-pentylnonyl behenate, 2-hexyldecyl behenate, 2-heptylundecyl behenate, 2-octyldodecyl behenate, isostearyl behenate, dodecyl 2-ethylhexanoate, myristyl 2-ethylhexanoate, palmityl 2-ethylhexanoate, stearyl 2-ethylhexanoate, 2-propylheptyl 2-ethylhexanoate, 2-butyloctyl 2-ethylhexanoate, isotridecyl 2-ethylhexanoate, 2-pentylnonyl 2-ethylhexanoate, 2-hexyldecyl 2-ethylhexanoate, 2-heptylundecyl 2-ethylhexanoate, 2-octyldodecyl 2-ethylhexanoate, isostearyl 2-ethylhexanoate, dodecyl isononanoate, myristyl isononanoate, palmityl isononanoate, stearyl isononanoate, behenyl isononanoate, 2-butyloctyl isononanoate, isotridecyl isononanoate, 2-pentylnonyl isononanoate, 2-hexyldecyl isononanoate, 2-heptylundecyl isononanoate, 2-octyldodecyl isononanoate, isostearyl isononanoate, decyl isodecanoate, undecyl isodecanoate, dodecyl isodecanoate, myristyl isodecanoate, palmityl isodecanoate, stearyl isodecanoate, 2-propylheptyl isodecanoate, 2-butyloctyl isodecanoate, isotridecyl isodecanoate, 2-pentylnonyl isodecanoate, 2-hexyldecyl isodecanoate, 2-heptylundecyl isodecanoate, 2-octyldodecyl isodecanoate, isostearyl isodecanoate, heptyl isotridecanoate, octyl isotridecanoate, nonyl isotridecanoate, decyl isotridecanoate, undecyl isotridecanoate, dodecyl isotridecanoate, myristyl isotridecanoate, palmityl isotridecanoate, stearyl isotridecanoate, isopropyl isotridecanoate, isobutyl isotridecanoate, 2-ethylhexyl isotridecanoate, 2-propylheptyl isotridecanoate, 2-butyloctyl isotridecanoate, isotridecyl isotridecanoate, 2-pentylnonyl isotridecanoate, 2-hexyldecyl isotridecanoate, 2-heptylundecyl isotridecanoate, 2-octyldodecyl isotridecanoate, isostearyl isotridecanoate, propyl isostearate, butyl isostearate, pentyl isostearate, hexyl isostearate, heptyl isostearate, octyl isostearate, nonyl isostearate, decyl isostearate, undecyl isostearate, dodecyl isostearate, myristyl isostearate, palmityl isostearate, stearyl isostearate, isopropyl isostearate, isobutyl isostearate, 2-ethylhexyl isostearate, 2-propylheptyl isostearate, 2-butyloctyl isostearate, isotridecyl isostearate, 2-pentylnonyl isostearate, 2-hexyldecyl isostearate, 2-heptylundecyl isostearate, 2-octyldodecyl isostearate, isostearyl isostearate, propyl oleate, butyl oleate, pentyl oleate, hexyl oleate, heptyl oleate, octyl oleate, nonyl oleate, decyl oleate, undecyl oleate, dodecyl oleate, myristyl oleate, palmityl oleate, stearyl oleate, isopropyl oleate, isobutyl oleate, 2-ethylhexyl oleate, 2-propylheptyl oleate, 2-butyloctyl oleate, isotridecyl oleate, 2-pentylnonyl oleate, 2-hexyldecyl oleate, 2-heptylundecyl oleate, 2-octyldodecyl oleate, isostearyl oleate, isononyl isononanoate, isodecyl isononanoate, isopropyl myristate, isopropyl palmitate, butyl stearate, hexyl laurate, myristyl myristate, hexyldecyl dimethyloctanoate, cetyl lactate, myristyl lactate, octyldodecyl lactate, lanolin acetate, cholesteryl 12-hydroxystearate, phytosteryl 12-hydroxystearate, phytosteryl oleate, ethylene glycol di-2-ethylhexanoate, dipentaerythritol fatty acid esters such as dipentaerythrityl hexa(hydroxystearate/stearate/rosinate) or dipentaerythrityl tetra(hydroxystearate/isostearate), alkyl glycol monoisostearate, neopentyl glycol diethylhexanoate, neopentyl glycol dicaprate, glyceryl di-2-heptylundecanoate, pentaerythritol tetra-2-ethylhexanoate, pentaerythritol tetraisostearate, glyceryl tri-2-ethylhexanoate, glyceryl tri(caprylate/caprate), glyceryl triisostearate, glyceryl tri(caprylate/caprate/myristate/stearate), glyceryl trimyristate, glyceryl tricaprylate, glyceryl tricaprate, glyceryl tri-2-heptylundecanoate, trimethylolpropane triisostearate, trimethylolpropane tri-2-ethylhexanoate, ditrimethylolpropane oligo ester(isostearate/sebacate), erythrityl triethylhexanoate, trehalose isostearate esters, dipentaerythrityl pentaisostearate, diglyceryl tetraisostearate, castor oil fatty acid methyl ester, fatty acid lanolin isopropyl ester, acetoglyceride, diisobutyl adipate, glycerin oligo ester (adipate. 2-ethylhexanoate • stearate), diglyceryl oligo ester (2-hexyldecanoate • sebacate), N-lanoloyl-L-glutamate-2-octyldodecyl ester, di-2-heptylundecyl adipate, hexyl laurate, ethyl laurate, 2-ethylhexyl sebacate, 2-hexyldecyl myristate, 2-hexyldecyl palmitate, 2-hexyldecyl adipate, diisopropyl sebacate, 2-ethylhexyl succinate, glyceryl triisopalmitate, ethyl acetate, butyl acetate, triethyl citrate, glyceryl tri(behenate/isostearate/eicosadioate), glyceryl (behenate/eicosadioate) and polyglyceryl (behenate/eicosadioate) .

Examples of animal and vegetable oils and fats include avocado oil, camellia oil, turtle oil, macadamia nut oil, corn oil, sunflower oil, mink oil, olive oil, rapeseed oil, egg yolk oil, sesame oil, persic oil, wheat germ oil, sasanqua oil, castor oil, linseed oil, safflower oil, grape seed oil, cottonseed oil, perilla oil, soybean oil, peanut oil, tea oil, kaya oil, rice bran oil, shinagiri oil, tung oil, jojoba oil, germ oil, primrose oil, cocoa butter, palm oil, beef tallow, mutton tallow, horse tallow, palm kernel oil, pork tallow, beef bone tallow, wax seed oil, neatsfoot tallow, wax, hydrogenated palm oil, hydrogenated palm oil, hydrogenated beef tallow, hydrogenated oil, hydrogenated castor oil, beeswax, candelilla wax, cotton wax, carnauba wax, bayberry wax, insect wax, spermaceti, montan wax, rice bran wax, kapok wax, sugar cane wax, lanolin, liquid lanolin, reduced lanolin, hydrogenated lanolin, jojoba wax and shellac wax.

Examples of silicone oils include linear polysiloxanes such as dimethyl polysiloxane, methyl phenyl polysiloxane or methyl hydrogen polysiloxane, cyclic polysiloxanes such as octamethyl cyclotetrasiloxane, decamethyl cyclopentasiloxane, dodecamethyl cyclohexasiloxane or tetrahydrotetramethyl cyclotetrasiloxane, and polyoxyethylene polyalkylsiloxane.

Examples of higher fatty acids include lauric acid, myristic acid, palmitic acid, stearic acid, behenic (beheninic) acid, oleic acid, undecylenic acid, tolic acid, isostearic acid, linoleic acid, linolenic acid, eicosapentaenoic acid (EPA) and docosahexaenoic acid (DHA).

Examples of higher alcohols include linear alcohols such as lauryl alcohol, cetyl alcohol, stearyl alcohol, behenyl alcohol, myristyl alcohol, oleyl alcohol or cetostearyl alcohol, and branched alcohols such as monostearyl glycerol ether (batyl alcohol), 2-decyltetradecinol, lanolin alcohol, cholesterol, phytosterol, hexyl dodecanol, isostearyl alcohol or octyl dodecanol.

Examples of phospholipids include soybean phospholipid, hydrogenated soybean phospholipid, rapeseed phospholipid, hydrogenated rapeseed phospholipid, egg yolk phospholipid and hydrogenated egg yolk phospholipid.

Examples of anionic surfactants include fatty acid soaps such as soap materials, sodium laurate or sodium palmitate, higher alkyl sulfate ester salts such as sodium lauryl sulfate or potassium lauryl sulfate, alkyl ether sulfate ester salts such as POE-lauryl sulfate triethanolamine or POE-sodium lauryl sulfate, N-acylsarcosinates such as sodium lauroyl sarcosinate, higher fatty acid amidosulfonates such as sodium N-myristoyl-N-methyl taurine, sodium palm oil fatty acid methyl taurine or sodium lauryl methyl tauride, phosphate ester salts such as sodium POE-oleyl ether phosphate or POE-stearyl ether phosphate, sulfosuccinates such as sodium di-2-ethylhexyl sulfosuccinate, sodium monolauroyl monoethanolamide polyoxyethylene sulfosuccinate or sodium lauryl polypropylene glycol sulfosuccinate, alkylbenzene sulfonates such as linear sodium dodecylbenzene sulfonate, linear triethanolamine dodecylbenzene sulfonate or linear dodecylbenzene sulfonic acid, N-acylglutamates such as monosodium N-lauroyl glutamate, disodium N-stearoyl glutamate or monosodium N-myristoyl-L-glutamate, higher fatty acid ester sulfate ester salts such as sodium hydrogenated palm oil fatty acid glycerin sulfate, sulfonated oils such as turkey red oil, POE-alkyl ether carboxylic acids, POE-alkyl allyl ether carboxylates, α-olefin sulfonates, higher fatty acid ester sulfonates, secondary alcohol sulfate ester salts, higher fatty acid alkyloyl amide sulfate ester salts, sodium lauroyl monoethanol amide succinates, N-palmitoyl aspartate ditriethanolamine and sodium caseinate.

Examples of cationic surfactants include alkyl trimethyl ammonium salts such as stearyl trimethyl ammonium chloride or lauryl trimethyl ammonium chloride, alkyl pyridinium salts such as poly(N,N'-dimethyl-3,5-methylenepyridinium) chloride or cetyl pyridinium chloride, alkyl quaternary ammonium salts, alkyl dimethyl benzyl ammonium salts, alkyl isoquinolinium salts, dialkyl morphonium salts, POE-alkyl amines, alkyl amine salts, polyamine fatty acid derivatives, amyl alcohol fatty acid derivatives, benzalkonium chloride, benzethonium chloride and organic modified clay minerals such as organic modified montmorillonite.

Examples of amphoteric surfactants include imidazoline-based amphoteric surfactants such as sodium 2-undecyl-N,N,N-(hydroxyethylcarboxymethyl)-2-imidazoline or sodium 2-cocoyl-2-imidazolinium hydroxide-1-carboxyethyloxy disodium salt, and betaine-based surfactants such as 2-heptadecyl-N-carboxymethyl-N-hydroxydiethyl imidazolinium betaine, lauryl dimethyl amino acetate betaine, alkyl betaines, amide betaines or sulfobetaines.

Examples of nonionic surfactants include POE-sorbitan fatty acid esters such as POE-sorbitan monooleate, POE-sorbitan monostearate, POE-sorbitan monooleate or POE-sorbitan tetraoleate, POE-sorbitol fatty acid esters such as POE-sorbitol tetraoleate, POE-sorbitol monooleate, POE-sorbitol pentaoleate or POE-sorbitol monostearate, POE-glycerin fatty acid esters such as POE-glycerin monostearate, POE-glycerin monoisostearate or POE-glycerin triisostearate, POE-fatty acid esters such as POE-monooleate, POE-distearate, POE-monodioleate or ethylene glycol stearate, POE-alkyl ethers such as POE-lauryl ether, POE-oleyl ether, POE-stearyl ether, POE-behenyl ether, POE-2-octyldodecyl ether or POE-cholestanol ether, POE/POP-alkyl ethers such as POE/POP-cetyl ether, POE/POP-2-decyltetradecyl ether, POE/POP-monobutyl ether, POE/POP-hydrogenated lanolin or POE/POP-glycerol ether, POE-castor oil derivatives or POE-hydrogenated castor oil derivatives such as POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester or POE-hydrogenated castor oil maleate, POE-beeswax-lanolin derivatives such as POE-sorbitol beeswax, alkanol amides such as palm oil diethanolamide, lauric acid monoethanolamide or fatty acid isopropanolamide, POE-propylene glycol fatty acid esters, POE-alkyl amines, POE-fatty acid amides, sucrose fatty acid esters, POE-nonylphenyl formaldehyde condensates, alkylethoxydimethylamine oxides, trioleyl phosphate, polyglycerin fatty acid esters such as polyglyceryl sesquicaprylate, polyglyceryl dicaprylate, polyglyceryl monolaurate, polyglyceryl monostearate, polyglyceryl monooleate, polyglyceryl distearate or polyglyceryl dioleate, modified silicones such as methyl polysiloxane-cetylmethyl polysiloxane-poly(oxyethylene-oxypropylene) methyl polysiloxane copolymer, sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate or diglycerol sorbitan penta-2-ethylhexylate, glycerin fatty acid esters such as cottonseed oil fatty acid monoglycerides, erucic acid monoglycerides, pyroglutamic acid glyceryl α,α-oleate, glyceryl monostearate or glyceryl monooleate, polyglycerin fatty acid esters such as diglyceryl monoisostearate, diglyceryl diisostearate, diglyceryl condensed ricinoleate or tetraglyceryl condensed ricinoleate, propylene glycol fatty acid esters such as propylene glycol monostearate, hydrogenated castor oil derivatives and glycerin alkyl ethers.

Examples of powdered components include inorganic powders such as talc, kaolin, mica, sericite, muscovite, phlogopite, synthetic mica, lepidolite, biotite, lithiamica, vermiculite, magnesium carbonate, calcium carbonate, aluminum silicate, barium silicate, calcium silicate, magnesium silicate, strontium silicate, metal tungstenates, magnesium, silica, zeolite, barium sulfate, calcined calcium sulfate (calcined gypsum), calcium phosphate, fluorapatite, hydroxyapatite, ceramic powders, metal soaps (such as zinc myristate, calcium palmitate or aluminum stearate) or boron nitride, organic powders such as polyamide resin powders (nylon powder), polyethylene powder, methyl polymethacrylate powder, polystyrene powder, styrene-acrylic acid copolymers, benzoguanamine resin powder, polytetrafluoroethylene powder or cellulose powder, inorganic white pigments such as titanium dioxide or zinc oxide, inorganic red pigments such as iron oxide (bengala) or iron titanate, inorganic brown pigments such as γ-iron oxide, inorganic yellow pigments such as yellow iron oxide or ocher, inorganic black pigments such as black iron oxide, carbon black or lower titanium oxides, inorganic violet pigments such as mango violet or cobalt violet, inorganic green pigments such as chromium oxide, chromium hydroxide or cobalt titanate, inorganic blue pigments such as ultramarine or Prussian blue, pearl pigments such as titanium oxide-coated mica, titanium oxide-coated bismuth oxychloride, titanium oxide-coated talc, colored titanium oxide-coated mica, bismuth oxychloride or fish scale guanine, metal powder pigments such as aluminum powder or copper powder, organic dyes such as red dye no. 201, red dye no. 202, red dye no. 204, red dye no. 205, red dye no. 220, red dye no. 226, red dye no. 228, red dye no. 405, orange dye no. 203, orange dye no. 204, yellow dye no. 205, yellow dye no. 401 or blue dye no. 404, zirconium, barium or aluminum lake organic dyes such as red dye no. 3, red dye no. 104, red dye no. 106, red dye no. 227, red dye no. 230, red dye no. 401, red dye no. 505, orange dye no. 205, yellow dye no. 4, yellow dye no. 5, yellow dye no. 202, yellow dye no. 203, green dye no. 3 or blue dye no. 1, and natural pigments such as chlorophyll or β-carotene. However, the powdered component is not limited to the aforementioned powdered components, and any powdered component can be used provided it can be applied to ordinary cosmetics.

Examples of moisturizers include polyethylene glycol, propylene glycol, glycerin, 1,3-butylene glycol, xylitol, sorbitol, maltitol, charonic acid, atelocollagen, sodium lactate, urea, bile acid salts, dl-pyrrolidone carboxylate, short-chain soluble collagen, diglycerin (EO) PO adducts (wherein, EO refers to ethylene oxide and PO refers to propylene oxide), rosa roxburghii fruit extract, yarrow extract and melilot extract.

Examples of natural water-soluble polymers include plant polymers such as gum arabic, tragacanth gum, galactan, guar gum, carob gum, karaya gum, carrageenan, pectin, agar, quince seed (marmelo), algal colloid (brown algae extract) or starch (including rice starch, cornstarch, potato starch and wheat starch), microbial polymers such as xanthan gum, dextran, succinoglucan or pullulan, and animal polymers such as collagen, casein, albumin or gelatin.

Examples of semi-synthetic water-soluble polymers include starch-based polymers such as carboxymethyl starch or methyl hydroxypropyl starch, cellulose-based polymers such as methyl cellulose, nitrocellulose, methyl hydroxypropyl cellulose, sodium cellulose sulfate, hydroxypropyl cellulose, hydroxypropyl methyl cellulose, carboxymethyl cellulose, sodium carboxymethyl cellulose, crystalline cellulose or powdered cellulose, and alginic acid-based polymers such as sodium alginate or propylene glycol alginate.

Examples of synthetic water-soluble polymers include vinyl-based polymers such as polyvinyl alcohol, polyvinyl methyl ether, polyvinyl pyrrolidone or carboxyvinyl polymer, polyoxyethylene-based polymers such as polyethylene glycol 20,000, 40,000 or 60,000, polyoxyethylene-polyoxypropylene copolymer-based polymers, acrylic-based polymers such as sodium polyacrylate, polyethylacrylate or polyacrylamide, polyethyleneimine and cationic polymers.

Examples of inorganic water-soluble polymers include bentonite, magnesium aluminum silicate (bee gum), laponite, hectorite and silicic anhydride.

Examples of ultraviolet absorbers include benzoic acid-based ultraviolet absorbers such as paraaminobenzoic acid (hereafter, abbreviated as "PABA"), PABA monoglycerin ester, N,N-dipropoxy-PABA ethyl ester, N,N-diethoxy-PABA ethyl ester, N,N-dimethyl-PABA ethyl ester, N,N-dimethyl-PABA butyl ester or N,N-dimethyl-PABA ethyl ester, anthranylic acid-based ultraviolet absorbers such as homomenthyl-N-acetyl anthranilate, salicylic acid-based ultraviolet absorbers such as amyl salicylate, menthyl salicylate, homomenthyl salicylate, octyl salicylate, phenyl salicylate, benzyl salicylate or p-isopropanol phenyl salicylate, cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p- methoxycinnamate, cyclohexyl-p-methoxycinnamate, ethyl-α-cyano-β-phenyl cinnamate, 2-ethylhexyl-α-cyano-β-phenyl cinnamate, or glyceryl mono-2-ethylhexanoyl- dipara-methoxycinnamate, benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4-phenylbenzophenone-2-carboxylate, 2-hydroxy-4-n-octoxybenzophenone or 4-hydroxy-3-carboxybenzophenone, 3-(4'-methylbenzylidene)-d,1-camphor, 3-benzylidene-d,1-camphor, urocanic acid, urocanic acid ethyl ester, 2-phenyl-5-methylbenzoxazole, 2,2'-hydroxy-5-methylphenylbenzotriazole, 2-(2'-hydroxy-5'-methylphenyl)benzotriazole, dibenzalazine, dianisoylmethane, 4-methoxy-4'-t-butyldibenzoylmethane, 5-(3,3-dimethyl-2-norbornylidene)-3-pentan-2-one and 2,4,6-trianilino-p-(carbo-2'-ethylhexyl-1'-oxy)1,3,5- triazine.

Examples of metal ion sequestering agents include 1-hydroxyethane-1,1-diphosphone, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid and trisodium ethylenediamine hydroxyethyl triacetate.

Examples of lower alcohols include methanol, ethanol, propanol, isopropanol, isobutyl alcohol and t-butyl alcohol.

Examples of polyvalent alcohols include divalent alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol or octylene glycol, trivalent alcohols such as glycerin, trimethylolpropane or 1,2,6-hexanetriol, tetravalent alcohols such as pentaerythritol, pentavalent alcohols such as xylitol, hexavalent alcohols such as sorbitol or mannitol, polyvalent alcohol polymers such as diethylene glycol, dipropylene glycol, triethylene glycol, polypropylene glycol, tetraethylene glycol, diglycerin, polyethylene glycol, triglycerin, tetraglycerin or polyglycerin, divalent alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methyl hexyl ether, ethylene glycol isoamyl ether, ethylene glycol benzyl ether, ethylene glycol isopropyl ether, ethylene glycol dimethyl ether, ethylene glycol diethyl ether or ethylene glycol dibutyl ether, divalent alcohol alkyl ethers such as diethylene glycol monomethyl ether, diethylene glycol monoethyl ether, diethylene glycol monobutyl ether, diethylene dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether or dipropylene glycol butyl ether, divalent alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate or propylene glycol monophenyl ether acetate, glycerin monoalkyl ethers such as xylyl alcohol, selachyl alcohol or batyl alcohol, sugar alcohols such as sorbitol, maltitol, maltotriose, mannitol, sucrose, erythritol, glucose, fructose, starch amylolysis sugar, maltose, xylitose, or alcohol prepared by reducing starch amylolysis sugar, glysolids, tetrahydrofurfuryl alcohol, POE-tetrahydrofurfuryl alcohol, POP-butyl ether, POP/POE-butyl ether, tripolyoxypropylene glycerol ether, POP-glycerol ether, POP-glycerol ether phosphoric acid and POP/POE-pentaneerythritol ether.

Examples of monosaccharides include trioses such as D-glyceryl aldehyde or dihydroxy acetone, tetroses such as D-erythrose, D-erythrulose, D-threose or erythritol, pentoses, such as L-arabinose, D-xylose, L-lyxose, D-arabinose, D-ribose, D-ribulose, D-xylulose or L-xylulose, hexoses such as D-glucose, D-talose, D-psicose, D-galactose, D-fructose, L-galactose, L-mannose or D-tagatose, heptoses, such as aldoheptose or hepturose, octoses such as octurose, deoxysaccharides such as 2-deoxy-D-ribose, 6-deoxy-L-galactose or 6-deoxy-L-mannose, aminosaccharides such as D-glucosamine, D-galactosamine, sialic acid, aminouronic acid or muramic acid, and uronic acids such as D-glucuronic acid, D-mannuronic acid, L-gluonic acid, D-galacturonic acid or L-iduronic acid.

Examples of oligosaccharides include sucrose, gunchianose, umbelliferose, lactose, planteose, isolignoses, α,α-trehalose, raffinose, lignoses, umbilicine, stachyose and belbascose.

Examples of polysaccharides include cellulose, chondroitin sulfuric acid, galactan, dermatan sulfate, glycogen, heparan sulfate, hyaluronic acid, keratan sulfate, chondroitin, mucoitin sulfate, guar gum, dextran, kerato sulfate, locust bean gum, succinoglucane and charonic acid.

Examples of amino acids include neutral amino acids such as threonine or cysteine, and basic amino acids such as hydroxylysine. Amino acid derivatives include sodium acylsarcosine (sodium lauroylsarcosine), acylglutamic acid salts, sodium acyl-β-alanine, glutathione and pyrrolidonecarboxylic acid.

Examples of organic amines include monoethanolamine, diethanolamine, triethanolamine, morpholine, triisopropanolamine, 2-amino-2-methyl-1,3-propanediol and 2-amino-2-methyl-1-propanol.

Examples of synthetic resin emulsions include acrylic resin emulsion, polyethyl acrylate emulsion, acrylic resin liquid, polyacrylalkyl ester emulsion and polyvinyl acetate resin emulsion.

Examples of pH regulators include buffers such as sodium hydroxide, potassium hydroxide, arginine, lactic acid/sodium lactate and citric acid/sodium citrate.

Examples of vitamins include vitamin A, B1, B2, B6, C, E and their derivatives, pantothenic acid and its derivatives, and biotin.

Examples of antioxidants include tocopherols, butylhydroxytoluene, butylhydroxyanisole and gallic acid esters.

Examples of antioxidant synergists include phosphoric acid, citric acid, ascorbic acid, maleic acid, malonic acid, succinic acid, fumaric acid, cephalin, hexametaphosphate, phytic acid and ethylenediamine-tetraacetic acid.

Examples of antiseptics include methyl parahydroxybenzoate, ethyl parahydroxybenzoate, butyl parahydroxybenzoate and phenoxyethanol. One type of these antiseptics may be used alone or two or more types may be used in combination.

Examples of salts include those obtained by a neutralization reaction between a strong acid and a strong base, such as sodium chloride, potassium chloride, sodium sulfate or magnesium sulfate.

Examples of fragrances include plant fragrances such as rose oil, jasmine oil or lavender oil, and synthetic fragrances such as limonene, citral, linalool or eugenol. One type of these fragrances may be used alone or two or more types may be used in combination.

Examples of other components that can be incorporated in addition to those described above include antiinflammatory agents such as glycyrrhizinic acid derivatives, glycyrrhetinic acid derivatives, salicylic acid derivatives, hinokitiol, zinc oxide or allantoin, whitening agents such as placenta extract or saxifrage extract, various extracts such as Phellodendron bark, Coptis japonica, Lithospermum erythrorhizon, Paeonia lactiflora, Swertia japonica, birch, sage, loquat, ginseng, aloe, Malva sylve, iris, grapes, dove wheat, luffa, lily, saffron, Cnidium officinale, shengjiang, Hypericum erectum, Ononis spinosa, garlic, red pepper, tangerine peel, Angelica acutiloba or seaweed, activators such as royal jelly, photosensitive agents, cholesterol derivatives or pediatric blood extract, blood circulation promoters such as nonylic acid vanillylamide, benzyl nicotinate, β-butoxyethyl nicotinate, capsaicin, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthine or γ-oryzanol, antiseborrheics such as sulfur or thiantol, and tranexamic acid, thiotaurine and hypotaurine.

There are no particular limitations on the method used to obtain the vaseline-like composition of the present invention, and the vaseline-like composition of the present invention can be obtained by, for example, warming and dissolving a mixture of one type or two or more types of components selected from the aforementioned components (A) and (B) and (C) and (E) and other of the aforementioned components as necessary, followed by stirring until uniform. In addition, mixtures obtained by preliminarily mixing each component either independently or as several types thereof may also be warmed and dissolved followed by kneading. Furthermore, the temperature during warming is preferably 80°C to 140°C.

The vaseline-like composition of the present invention can be preferably used as a raw material of a cosmetic, quasi drug or pharmaceutical and the like. Furthermore, the vaseline-like composition of the present invention may also be directly as a cosmetic, quasi drug or pharmaceutical and the like.

In the case of using the vaseline-like composition of the present invention as a type of raw material of a cosmetic, the content of the vaseline-like composition of the present invention in the cosmetic is 0.1% by weight to 95% by weight, preferably 0.5% by weight to 90% by weight, more preferably 1% by weight to 80% by weight, and even more preferably 5% by weight to 50% by weight.

A target product can be produced in accordance with ordinary methods by incorporating various types of components typically used in cosmetics, quasi drugs or pharmaceuticals and the like in a cosmetic containing the vaseline-like composition as necessary within a range that does not impair the effects of the present invention in addition to the vaseline-like composition of the present invention. For example, a finished product can be produced in accordance with ordinary methods corresponding to the target drug form by suitably incorporating water, oily components other than oily components serving as essential components of the present invention, anionic surfactants, cationic surfactants, amphoteric surfactants, nonionic surfactants, powdered components, moisturizers, natural water-soluble polymers, semi-synthetic water-soluble polymers, synthetic water-soluble polymers, inorganic water-soluble polymers, ultraviolet absorbers, metal ion sequestering agents, lower alcohols, polyvalent alcohols, monosaccharides, oligosaccharides, amino acids, organic amines, synthetic resin emulsions, pH regulators, vitamins, antioxidants, antioxidant synergists, antiseptics, salts or fragrances as necessary. Examples of these components include the same components as those previously described and microcrystalline wax for the oily component.

A method conventionally used to produce cosmetics is used as a production method for obtaining the cosmetic of the present invention without requiring any special means or devices. For example, in the case of producing a emulsified cosmetic, an oily phase containing the vaseline-like composition of the present invention and an aqueous phase are respectively warmed to 60°C to 80°C, and while in this state, the aqueous phase is gradually added to the oily phase to carry out preliminary emulsification, and the emulsion particles are uniformly mixed with a homomixer followed by deaeration, filtering and cooling. In addition to the vaseline-like composition of the present invention, an oily phase having oily components in various contents and the like can be used for the oily phase. In addition, a moisturizer can be added to purified water for use as the aqueous phase.

In the case of producing a solid cosmetic such as lipstick, a cosmetic composition containing the vaseline-like composition of the present invention, a liquid oil, an oily component such as wax, and a powder such as pigment are heated and dissolved at 80°C to 120°C followed by suitably mixing to uniformity with a three-roller mixer, pouring into a metal mold and the like, and cooling.

The cosmetic of the present invention can be made to be in a desired form, such as an oil-dissolved cosmetic, emulsified cosmetic such as an O/W, W/O, W/O/W or O/W/O cosmetic, solubilized cosmetic or solid cosmetic. In addition, examples of types of cosmetics include makeup cosmetics such as cleansing oil, milky lotion, cream, beauty wash, lipstick, foundation or eye shadow, and hair care cosmetics such as hair wax, hair spray or hair conditioner.

### [Examples]

Although the following provides a more detailed explanation of the present invention by indicating examples and comparative examples thereof, the present invention is not limited thereto. Furthermore, in the following examples and comparative examples, the term "percent (%)" refers to "% (percent) by weight" unless specifically indicated otherwise.

### Synthesis Examples 1 to 3 (Poly(12-hydroxystearic acid))

### (Synthesis Example 1)

500 g of 12-hydroxystearic acid (Kawaken Fine Chemicals Co., Ltd., trade name: Hydroxystearic Acid) were charged into a 1 L four-necked flask equipped with a stirrer, thermometer, nitrogen gas inlet tube and moisture separator, followed by the addition of 0.5 g of tin chloride as a catalyst and reacting for 15 hours at a temperature of 200°C in the presence of flowing nitrogen while removing water formed during the reaction. Following completion of the reaction, the catalyst was filtered out, and after decolorizing using activated charcoal, deodorization was carried out by blowing in water vapor under reduced pressure. When the acid value of the resulting polymer was measured, the poly(12-hydroxystearic acid) of Synthesis Example 1 was obtained in which the acid value was 33 mgKOH/g, the average molecular weight as calculated from the acid value was 1700, and the average degree of polymerization as calculated from the average molecular weight was 6 (yield: 430 g).

### (Synthesis Example 2)

500 g of 12-hydroxystearic acid (Kokura Synthetic Industries, Ltd., trade name: 12-Hydroxystearic Acid) were charged into a 1 L four-necked flask equipped with a stirrer, thermometer, nitrogen gas inlet tube and moisture separator, followed by the addition of tin chloride as a catalyst in an amount equal to 0.1% of the total charged amount and reacting for 15 hours at a temperature of 200°C in the presence of flowing nitrogen while removing water formed during the reaction. Following completion of the reaction, the catalyst was filtered out, and after decolorizing using activated charcoal, deodorization was carried out by blowing in water vapor under reduced pressure. When the acid value of the resulting polymer was measured, the poly(12-hydroxystearic acid) of Synthesis Example 2 was obtained in which the acid value was 20 mgKOH/g, the average molecular weight as calculated from the acid value was 2820, and the average degree of polymerization as calculated from the average molecular weight was 10 (yield: 410 g).

### (Synthesis Example 3)

500 g of 12-hydroxystearic acid (Kokura Synthetic Industries, Ltd., trade name: 12-Hydroxystearic Acid) were charged into a 1 L four-necked flask equipped with a stirrer, thermometer, nitrogen gas inlet tube and moisture separator, followed by the addition of tin chloride as a catalyst in an amount equal to 1% of the total charged amount and reacting for 20 hours at a temperature of 200°C in the presence of flowing nitrogen while removing water formed during the reaction. Following completion of the reaction, the catalyst was filtered out, and after decolorizing using activated charcoal, deodorization was carried out by blowing in water vapor under reduced pressure. When the acid value of the resulting polymer was measured, the poly(12-hydroxystearic acid) of Synthesis Example 3 was obtained in which the acid value was 14 mgKOH/g, the average molecular weight as calculated from the acid value was 4008, and the average degree of polymerization as calculated from the average molecular weight was 14 (yield: 400 g).

### Synthesis Examples 4 to 6 (Poly(12-hydroxystearic acid) Derivatives)

### (Synthesis Example 4)

386 g (0.227 moles) of the poly(12-hydroxystearic acid) obtained in Synthesis Example 1 and 14 g (0.055 moles) of dipentaerythritol (Koei Perstorp Co., Ltd., trade name: "Di-Pentaerit") were added to a 1L four-necked flask equipped with a stirrer, thermometer, nitrogen gas inlet tube and moisture separator, followed by the addition of 0.4 g of tin chloride as a catalyst and reacting at a temperature of 210°C in the presence of flowing nitrogen while removing water formed during the reaction until the acid value of the product reached 1 mgKOH/g or less. Following completion of the reaction, the catalyst was filtered out, and after decolorizing using activated clay, deodorization was carried out by blowing in water vapor under reduced pressure to obtain 340 g of the poly(12-hydroxystearic acid) derivative of Synthesis Example 4 (acid value: 0.5 mgKOH/g, hydroxyl value: 35 mgKOH/g).

### (Synthesis Example 5)

386 g (0.137 moles) of the poly(12-hydroxystearic acid) obtained in Synthesis Example 2 and 14 g (0.055 moles) of dipentaerythritol (Koei Perstorp Co., Ltd., trade name: "Di-Pentaerit") were added to a 1 L four-necked flask equipped with a stirrer, thermometer, nitrogen gas inlet tube and moisture separator, followed by the addition of 0.4 g of tin chloride as a catalyst and reacting at a temperature of 210°C in the presence of flowing nitrogen while removing water formed during the reaction until the acid value of the product reached 1 mgKOH/g or less. Following completion of the reaction, the catalyst was filtered out, and after decolorizing using activated clay, deodorization was carried out by blowing in water vapor under reduced pressure to obtain 340 g of the poly(12-hydroxystearic acid) derivative of Synthesis Example 5 (acid value: 0.8 mgKOH/g, hydroxyl value: 45 mgKOH/g).

### (Synthesis Example 6)

386 g (0.096 moles) of the poly(12-hydroxystearic acid) obtained in Synthesis Example 3 and 14 g (0.055 moles) of dipentaerythritol (Koei Perstorp Co., Ltd., trade name: "Di-Pentaerit") were added to a 1L four-necked flask equipped with a stirrer, thermometer, nitrogen gas inlet tube and moisture separator, followed by the addition of 0.4 g of tin chloride as a catalyst and reacting at a temperature of 210°C in the presence of flowing nitrogen while removing water formed during the reaction until the acid value of the product reached 1 mgKOH/g or less. Following completion of the reaction, the catalyst was filtered out, and after decolorizing using activated clay, deodorization was carried out by blowing in water vapor under reduced pressure to obtain 340 g of the poly(12-hydroxystearic acid) derivative of Synthesis Example 6 (acid value: 0.9 mgKOH/g, hydroxyl value: 50 mgKOH/g).

### Synthesis Examples 7 and 8 (Dimer Dilinoleic Acid Derivative)

### (Synthesis Example 7) Phytosteryl/Isostearyl Dimer Dilinoleate

172 g (0.3 moles) of dimer dilinoleic acid (Unichema Corp., Pripol 1006), 79 g (0.2 moles) of phytosterol (Tama Biochemical Co., Ltd.), 50 g of heptane and 0.5 g of para-toluene sulfonic acid were added to a 1 L four-necked flask equipped with a stirrer, thermometer, nitrogen gas inlet tube and moisture separator, followed by heating to within the range of 100°C to 110°C in the presence of flowing nitrogen and reacting for 8 hours while removing water formed during the reaction. Subsequently, 108 g (0.4 moles) of isostearyl alcohol (Sasol Inc., Isofol 18E) were further added and allowed to react for 10 hours. After cooling, the reaction mixture was diluted with heptane and unreacted carboxylic acid was removed by washing with aqueous sodium hydroxide solution. The solvent was then distilled off under reduced pressure to obtain 320 g of the dimer dilinoleic acid derivative (phytosteryl/isostearyl dimer dilinoleate) of Synthesis Example 7 (yield: 90%). In the dimer dilinoleic acid derivative of Synthesis Example 7, the molar ratio of phytosterol to the total amount of ester-bonded alcohol per 1 molecule of dimer dilinoleic acid was 0.33.

### (Synthesis Example 8) Cholesteryl/Stearyl/Cetyl Dimer Dilinoleate

172 g (0.3 moles) of dimer dilinoleic acid (Unichema Corp., Pripol 1006), 79 g (0.2 moles) of cholesterol, 50 g of heptane and 0.5 g of para-toluene sulfonic acid were added to a 1 L four-necked flask equipped with a stirrer, thermometer, nitrogen gas inlet tube and moisture separator, followed by heating to 100°C to within the range of 110°C in the presence of flowing nitrogen and reacting for 8 hours while removing water formed during the reaction. Subsequently, 54 g (0.2 moles) of stearyl alcohol and 48 g (0.2 moles) of cetyl alcohol were further added and allowed to react for 10 hours. After cooling, the reaction mixture was diluted with heptane and unreacted carboxylic acid was removed by washing with aqueous sodium hydroxide solution. The solvent was then distilled off under reduced pressure to obtain 270 g of the dimer dilinoleic acid derivative (cholesteryl/stearyl/cetyl dimer dilinoleate) of Synthesis Example 8 (yield: 90%). In the dimer dilinoleic acid derivative of Synthesis Example 8, the molar ratio of cholesterol to the total amount of ester-bonded alcohol 1 per molecule of dimer dilinoleic acid was 0.33.

### Vaseline-like Compositions (Examples 1 to 28 and Comparative Examples 1 to 35)

### [Sample Preparation Method]

Each component was weighed out according to the incorporated amounts indicated in Tables 1 to 10, and after warming and dissolving at 110°C and stirring to uniformity, the mixtures were cooled to room temperature while continuing to stir to obtain vaseline-like compositions of Examples 1 to 28 and Comparative Examples 1 to 35.

### [Evaluation of Moisture Occlusion]

Moisture occlusion was evaluated by observing changes in electrical conductivity of the horny layer of skin before and after applying the samples using the "Skicon 200" manufactured by I.B.S. Co., Ltd. The "Skicon 200" measures electrical conductivity of the horny layer by applying a high-frequency voltage to the horny layer, and an increase in electrical conductivity is measured when the moisture content of the horny layer is high. Namely, when a sample having high moisture occlusion is applied to the skin, moisture transpiration is inhibited, thereby resulting in an increase in skin moisture content and causing an increase in electrical conductivity to be measured as compared with prior to application of the sample.

A mark covering an area of 2 cm × 2 cm was made on the insides of the forearms of 10 panelists followed by measurement of electrical conductivity of the horny layer before sample application. 20 µL of each sample were spread out on the skin and allowed to stand for 2 hours, followed by wiping off the sample with a cotton swab soaked in hexane. Electrical conductivity of the horny layer was measured with the "Skicon 200" immediately thereafter to measure the change in electrical conductivity following sample application. Electrical conductivity after sample application of 400% or more was assigned a score of 2, that of 200% to 400% was assigned a score of 1 and that of less than 200% was assigned a score of 0 based on a value of 100% for electrical conductivity prior to sample application, and moisture occlusion was evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

### [Evaluation of Skin Spreadability]

Ten panelists were made to apply each sample to the skin to obtain a sensory evaluation of skin spreadability. Vaseline (Nisshin OilliO Group, Ltd., "Nomcoat W") was used as a comparative sample, skin spreadability more favorable than that of the comparative sample was assigned a score of 2, that equal to spreadability of the comparative sample was assigned a score of 1, and that worse than spreadability of the comparative sample was assigned a score of 0, and skin spreadability was evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

### [Evaluation of Stickiness]

Ten panelists were made to apply each sample to the skin to obtain a sensory evaluation of stickiness. Vaseline (Nisshin OilliO Group, Ltd., "Nomcoat W") was used as a comparative sample, stickiness less than that of the comparative sample was assigned a score of 2, that equal to stickiness of the comparative sample was assigned a score of 1, and stickiness greater than that of the comparative sample was assigned a score of 0, and stickiness was evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

### [Evaluation of Skin Blendability]

Ten panelists were made to apply each sample to the skin to obtain a sensory evaluation of skin blendability. Vaseline (Nisshin OilliO Group, Ltd., "Nomcoat W") was used as a comparative sample, skin blendability more favorable than that of the comparative sample was assigned a score of 2, that equal to blendability of the comparative sample was assigned a score of 1, and that worse than blendability of the comparative sample was assigned a score of 0, and skin blendability was evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

**[Table 1]**

| Examples 1 to 5 | | (wt%) | | | | |
|---|---|---|---|---|---|---|
| Table 1 | | Ex. 1 | Ex. 2 | Ex. 3 | Ex. 4 | Ex. 5 |
| Diisostearyl malate^{*1} | | 80 | 80 | 80 | 80 | 80 |
| Polyethylene wax^{*2} | | 10 | - | - | 10 | 10 |
| Ceresin wax^{*3} | | - | 10 | - | - | - |
| Paraffin wax^{*4} | | - | - | 10 | - | - |
| Compound of Synthesis Example 1 | | 10 | 10 | 10 | - | - |
| Compound of Synthesis Example 2 | | - | - | - | 10 | - |
| Compound of Synthesis Example 3 | | - | - | - | - | 10 |
| Evaluation | Moisture occlusion | ○ | ○ | ○ | ○ | ○ |
| | Spreadability | ⊚ | ⊚ | ⊚ | ⊚ | ○ |
| | Stickiness | ⊚ | ○ | ○ | ○ | ○ |
| | Blendability | ○ | ○ | ○ | ○ | ○ |

| | | | | | | |
|---|---|---|---|---|---|---|
| *1: "Cosmol 222" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | | |

**[Table 2]**

| Examples 6 to 10 | | (wt%) | | | | |
|---|---|---|---|---|---|---|
| Table 2 | | Ex. 6 | Ex. 7 | Ex. 8 | Ex. 9 | Ex. 10 |
| Diisostearyl malate^{*1} | | 80 | 80 | 80 | 80 | 80 |
| Polyethylene wax^{*2} | | 10 | - | - | 10 | 10 |
| Ceresin wax^{*3} | | - | 10 | - | - | - |
| Paraffin wax^{*4} | | - | - | 10 | - | - |
| Compound of Synthesis Example 4 | | 10 | 10 | 10 | - | - |
| Compound of Synthesis Example 5 | | - | - | - | 10 | - |
| Compound of Synthesis Example 6 | | - | - | - | - | 10 |
| Evaluation | Moisture occlusion | ⊚ | ⊚ | ⊚ | ⊚ | ⊚ |
| | Spreadability | ⊚ | ⊚ | ⊚ | ⊚ | ⊚ |
| | Stickiness | ⊚ | ⊚ | ⊚ | ⊚ | ⊚ |
| | Blendability | ⊚ | ⊚ | ⊚ | ⊚ | ⊚ |

| | | | | | | |
|---|---|---|---|---|---|---|
| *1: "Cosmol 222" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | | |

**[Table 3]**

| Examples 11 to 14 | | (wt%) | | | |
|---|---|---|---|---|---|
| Table 3 | | Ex. 11 | Ex. 12 | Ex. 13 | Ex. 14 |
| Diisostearyl malate^{*1} | | 80 | 80 | 80 | 80 |
| Polyethylene wax^{*2} | | 10 | - | - | 10 |
| Ceresin wax^{*3} | | - | 10 | - | - |
| Paraffin wax^{*4} | | - | - | 10 | - |
| Compound of Synthesis Example 7 | | 10 | 10 | 10 | - |
| Compound of Synthesis Example 8 | | - | - | - | 10 |
| Evaluation | Moisture occlusion | ⊚ | ⊚ | ⊚ | ⊚ |
| | Spreadability | ⊚ | ⊚ | ○ | ⊚ |
| | Stickiness | ○ | ○ | ○ | ○ |
| | Blendability | ⊚ | ⊚ | ⊚ | ⊚ |

| | | | | | |
|---|---|---|---|---|---|
| *1: "Cosmol 222" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | |

**[Table 4]**

| Examples 15 to 19 | | (wt%) | | | | |
|---|---|---|---|---|---|---|
| Table 4 | | Ex. 15 | Ex. 16 | Ex. 17 | Ex. 18 | Ex. 19 |
| Diglyceryl triisostearate^{*1} | | 80 | 80 | 80 | 80 | 80 |
| Polyethylene wax^{*2} | | 10 | - | - | 10 | 10 |
| Ceresin wax^{*3} | | - | 10 | - | - | - |
| Paraffin wax^{*4} | | - | - | 10 | - | - |
| Compound of Synthesis Example 1 | | 10 | 10 | 10 | - | - |
| Compound of Synthesis Example 2 | | - | - | - | 10 | - |
| Compound of Synthesis Example 3 | | - | - | - | - | 10 |
| Evaluation | Moisture occlusion | ○ | ○ | ○ | ○ | ○ |
| | Spreadability | ○ | ○ | ○ | ○ | ○ |
| | Stickiness | ○ | ○ | ○ | ○ | ○ |
| | Blendability | ⊚ | ⊚ | ⊚ | ⊚ | ⊚ |

| | | | | | | |
|---|---|---|---|---|---|---|
| *1: "Cosmol 43V" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | | |

**[Table 5]**

| Tables 20 to 24 | | (wt%) | | | | |
|---|---|---|---|---|---|---|
| Table 5 | | Ex.20 | Ex. 21 | Ex.22 | Ex. 23 | Ex. 24 |
| Diglyceryl triisostearate^{*1} | | 80 | 80 | 80 | 80 | 80 |
| Polyethylene wax^{*2} | | 10 | - | - | 10 | 10 |
| Ceresin wax^{*3} | | - | 10 | - | - | - |
| Paraffin wax^{*4} | | - | - | 10 | - | - |
| Compound of Synthesis Example 4 | | 10 | 10 | 10 | - | - |
| Compound of Synthesis Example 5 | | - | - | - | 10 | - |
| Compound of Synthesis Example 6 | | - | - | - | - | 10 |
| Evaluation | Moisture occlusion | ○ | ○ | ○ | ○ | ○ |
| | Spreadability | ⊚ | ⊚ | ⊚ | ⊚ | ⊚ |
| | Stickiness | ⊚ | ⊚ | ⊚ | ⊚ | ⊚ |
| | Blendability | ⊚ | ⊚ | ⊚ | ⊚ | ⊚ |

| | | | | | | |
|---|---|---|---|---|---|---|
| *1: "Cosmol 43V" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | | |

**[Table 6]**

| Examples 25 to 28 | | (wt%) | | | |
|---|---|---|---|---|---|
| Table 6 | | Ex. 25 | Ex.26 | Ex.27 | Ex.28 |
| Diglyceryl triisostearate^{*1} | | 80 | 80 | 80 | 80 |
| Polyethylene wax^{*2} | | 10 | - | - | 10 |
| Ceresin wax^{*3} | | - | 10 | - | - |
| Paraffin wax^{*4} | | - | - | 10 | - |
| Compound of Synthesis Example 7 | | 10 | 10 | 10 | - |
| Compound of Synthesis Example 8 | | - | - | - | 10 |
| Evaluation | Moisture occlusion | ○ | ○ | ○ | ○ |
| | Spreadability | ⊚ | ⊚ | ○ | ⊚ |
| | Stickiness | ○ | ○ | ○ | ○ |
| | Blendability | ⊚ | ⊚ | ⊚ | ⊚ |

| | | | | | |
|---|---|---|---|---|---|
| *1: "Cosmol 43V" (Nisshin OilliO Group, Ltd.) *2: "Performaline PL" (Nikko Chemicals Co., Ltd.) *3: "Ceresin 810N" (Nikko Rica Corp.) *4: "Paraffin 155F" (Nippon Seiro Co., Ltd.) | | | | | |

**[Table 7]**

| Comparative Examples 1 to 10 | | | | | | | | | (wt%) | | |
|---|---|---|---|---|---|---|---|---|---|---|---|
| Table 7 | | Comp. Ex.1 | Comp. Ex.2 | Comp. Ex.3 | Comp. Ex.4 | Comp. Ex.5 | Comp. Ex.6 | Comp. Ex.7 | Comp. Ex.8 | Comp. Ex.9 | Comp. Ex.10 |
| Diisostearyl malate^{*1} | | - | 90 | 90 | - | 90 | 90 | - | 90 | 90 | - |
| Polyethylene wax^{*2} | | - | - | 10 | 60 | - | 10 | 60 | - | 10 | 60 |
| Compound of Synthesis Example 1 | | - | 10 | - | 40 | - | - | - | - | - | - |
| Compound of Synthesis Example 4 | | - | - | - | - | 10 | - | 40 | - | - | - |
| Compound of Synthesis Example 7 | | - | - | - | - | - | - | - | 10 | - | 40 |
| vase | | 100 | - | - | - | - | - | - | - | - | - |
| Evaluation | Moisture occlusion | ⊚ | × | ○ | × | × | ○ | Δ | × | ○ | ×× |
| | Spreadability | Δ | ○ | × | ○ | ⊚ | × | × | ○ | × | ×× |
| | Stickiness | Δ | × | × | × | × | × | × | × | × | × |
| | Blendability | Δ | O | Δ | × | ○ | Δ | × | ○ | Δ | ×× |

| | | | | | | | | | | | |
|---|---|---|---|---|---|---|---|---|---|---|---|
| *1: "Cosmol 222" (Nisshin OilliO Group, Ltd.) *2: "Performaline PL" (Nikko Chemicals Co., Ltd.) *3: "Nomcoat W" (Nisshin OilliO Group, Ltd.) | | | | | | | | | | | |

**[Table 8]**

| Comparative Examples 11 to 18 | | | | | | | (wt%) | | |
|---|---|---|---|---|---|---|---|---|---|
| Table 8 | | Comp. Ex. 11 | Comp. Ex. 12 | Comp. Ex. 13 | Comp. Ex. 14 | Comp. Ex. 15 | Comp. Ex. 16 | Comp. Ex. 17 | Comp. Ex. 18 |
| Diisostearyl malate^{*1} | | 92 | 35 | 86 | 65 | 86 | 65 | 86 | 65 |
| Polyethylene wax^{*2} | | 4 | 55 | 10 | 10 | 10 | 10 | 10 | 10 |
| Compound of Synthesis Example 1 | | - | - | 4 | 25 | - | - | - | - |
| Compound of Synthesis Example 4 | | 4 | 10 | - | - | 4 | 25 | - | - |
| Compound of Synthesis Example 7 | | - | - | - | - | - | - | 4 | 25 |
| Evaluation | Moisture occlusion | × | ×× | ○ | × | ○ | × | ○ | × |
| | Spreadability | ○ | ×× | × | Δ | Δ | Δ | × | Δ |
| | Stickiness | × | × | × | × | × | × | × | Δ |
| | Blendability | ○ | ×× | Δ | Δ | Δ | Δ | Δ | Δ |

| | | | | | | | | | |
|---|---|---|---|---|---|---|---|---|---|
| *1: "Cosmol 222" (Nisshin OilliO Group, Ltd.) *2: "Performaline PL" (Nikko Chemicals Co., Ltd.) | | | | | | | | | |

**[Table 9]**

| Comparative Examples 19 to 27 | | | | | | | | (wt%) | | |
|---|---|---|---|---|---|---|---|---|---|---|
| Table 9 | | Comp. Ex. 19 | Comp. Ex. 20 | Comp. Ex. 21 | Comp. Ex. 22 | Comp. Ex. 23 | Comp. Ex. 24 | Comp. Ex. 25 | Comp. Ex. 26 | Comp. Ex. 27 |
| Diglyceryl diisostearate ^{*1} | | 90 | 90 | - | 90 | 90 | - | 90 | 90 | - |
| Polyethylene wax^{*2} | | - | 10 | 60 | - | 10 | 60 | - | 10 | 60 |
| Compound of Synthesis Example 1 | | 10 | - | 40 | - | - | - | - | - | - |
| Compound of Synthesis Example 4 | | - | - | - | 10 | - | 40 | - | - | - |
| Compound of Synthesis Example 7 | | - | - | - | - | - | - | 10 | - | 40 |
| Evaluation | Moisture occlusion | × | ○ | × | × | ○ | × | × | ○ | ×× |
| | Spreadability | ○ | Δ | ○ | ⊚ | × | × | ○ | × | ×× |
| | Stickiness | × | × | × | × | × | × | × | × | × |
| | Blendability | Δ | Δ | × | Δ | Δ | × | Δ | Δ | ×× |

| | | | | | | | | | | |
|---|---|---|---|---|---|---|---|---|---|---|
| *1: "Cosmol 43V" (Nisshin OilliO Group, Ltd.) *2: "Performaline PL" (Nikko Chemicals Co., Ltd.) | | | | | | | | | | |

**[Table 10]**

| Comparative Examples 28 to 35 | | | | | | | (wt%) | | |
|---|---|---|---|---|---|---|---|---|---|
| Table 10 | | Comp. Ex. 28 | Comp. Ex. 29 | Comp. Ex. 30 | Comp. Ex. 31 | Comp. Ex. 32 | Comp. Ex. 33 | Comp. Ex. 34 | Comp. Ex. 35 |
| Diglyceryl diisostearate^{*1} | | 92 | 35 | 86 | 65 | 86 | 65 | 86 | 65 |
| Polyethylene wax^{*2} | | 4 | 55 | 10 | 10 | 10 | 10 | 10 | 10 |
| Compound of Synthesis Example 1 | | - | - | 4 | 25 | - | - | - | - |
| Compound of Synthesis Example 4 | | 4 | 10 | - | - | 4 | 25 | - | - |
| Compound of Synthesis Example 7 | | - | - | - | - | - | - | 4 | 25 |
| Evaluation | Moisture occlusion | × | ×× | ○ | × | ○ | × | ○ | × |
| | Spreadability | ○ | ×× | × | Δ | Δ | Δ | × | × |
| | Stickiness | × | × | × | × | × | × | × | Δ |
| | Blendability | Δ | ×× | × | Δ | Δ | × | Δ | Δ |

| | | | | | | | | | |
|---|---|---|---|---|---|---|---|---|---|
| *1: "Cosmol 43V" (Nisshin OilliO Group, Ltd.) *2: "Performaline PL" (Nikko Chemicals Co., Ltd.) | | | | | | | | | |

As shown in Tables 1 to 10, the vaseline-like composition of the present invention was clearly determined to have little stickiness and superior skin blendability while having moisture occlusion and skin spreadability comparable to vaseline (Comparative Example 1). Comparative Examples 1 to 35 lacked some of the constituents of the present invention, and were unable to simultaneously realize moisture occlusion, skin spreadability, absence of stickiness and skin blendability.

### Emollient Cream (Examples 29 to 32, Comparative Example 36)

Components (A) were warmed and dissolved at 80°C in the incorporated amounts shown in Table 11, and the components (B) also warmed and dissolved at 80°C were slowly added thereto while stirring at 2000 rpm with a disperser. After cooling to 50°C while continuing to stir, component (C) was added and stirred to uniformity followed by cooling to room temperature and degassing to obtain an emollient cream.

### [Evaluation of Moisture Occlusion]

Moisture occlusion was evaluated in the same manner as Examples 1 to 28 and Comparative Examples 1 to 35.

### [Sensory Evaluations]

Ten panelists were made to apply each sample to the skin to evaluate skin spreadability, stickiness and skin blendability. Favorable results were assigned a score of 2, average results were assigned a score of 1, and poor results were assigned a score of 0, and the parameters were evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

**[Table 11]**

| Emollient Cream Formulation and Evaluation Results (wt%) | | | | | | |
|---|---|---|---|---|---|---|
| Table 11 | Components | Ex. 29 | Ex. 30 | Ex. 31 | Ex. 32 | Comp. Ex. 36 |
| (A) | Vaseline-like composition of Example 6 | 10.0 | - | - | - | - |
| | Vaseline-like composition of Example 11 | - | 10.0 | - | - | - |
| | Vaseline-like composition of Example 20 | - | - | 10.0 | - | - |
| | Vaseline-like composition of Example 25 | - | - | - | 10.0 | - |
| | Vaseline^{*1} | - | - | - | - | 10.0 |
| | Olive oil^{*2} | 5.0 | 5.0 | 5.0 | 5.0 | 5.0 |
| | Squalane | 2.0 | 2.0 | 2.0 | 2.0 | 2.0 |
| | Glyceryl tri-2-ethyl hexanoate^{*3} | 2.0 | 2.0 | 2.0 | 2.0 | 2.0 |
| | Dipentaerythrityl hexa(hydroxystearate/ stearate/rosinate) ^{*4} | 4.0 | 4.0 | 4.0 | 4.0 | 4.0 |
| | Microcrystalline wax | 1.0 | 1.0 | 1.0 | 1.0 | 1.0 |
| | Cetanol | 3.0 | 3.0 | 3.0 | 3.0 | 3.0 |
| | Polyglyceryl oleate-10^{*5} | 1.2 | 1.2 | 1.2 | 1.2 | 1.2 |
| | Polyglyceryl oleate-2^{*6} | 0.3 | 0.3 | 0.3 | 0.3 | 0.3 |
| | Stearic acid | 0.5 | 0.5 | 0.5 | 0.5 | 0.5 |
| | Glyceryl monostearate^{*7} | 0.5 | 0.5 | 0.5 | 0.5 | 0.5 |
| (B) | Glycerin | 5.0 | 5.0 | 5.0 | 5.0 | 5.0 |
| | 1,3-butylene glycol | 10.0 | 10.0 | 10.0 | 10.0 | 10.0 |
| | Methyl parahydroxybenzoate | 0.1 | 0.1 | 0.1 | 0.1 | 0.1 |
| | Sodium hydroxide (1% aqueous solution) | 3.9 | 3.9 | 3.9 | 3.9 | 3.9 |
| | Ion exchange water | 36.5 | 30.5 | 30.5 | 30.5 | 30.5 |
| (C) | Carboxyvinyl polymer^{*8} (1% aqueous solution) | 15.0 | 15.0 | 15.0 | 15.0 | 15.0 |
| Evaluation | Moisture occlusion | ⊚ | ⊚ | ⊚ | ⊚ | ⊚ |
| | Spreadability | ⊚ | ⊚ | ⊚ | ⊚ | ⊚ |
| | Stickiness | ⊚ | ○ | ⊚ | ○ | ×× |
| | Blendability | ⊚ | ⊚ | ⊚ | ⊚ | ×× |

| | | | | | | |
|---|---|---|---|---|---|---|
| *1: "Nomcoat W" (Nisshin OilliO Group, Ltd.) *2: "Refined Olive Oil" (Yokozeki Oil & Fat Industries, Co., Ltd.) *3: "T.I.O." (Nisshin OilliO Group, Ltd.) *4: "Cosmol 168ARV" (Nisshin OilliO Group, Ltd.) *5: "Saracos PG-180" (Nisshin OilliO Group, Ltd.) *6: "Saracos DG-180" (Nisshin OilliO Group, Ltd.) *7: "Poem S-100" (Riken Vitamin Co., Ltd.) *8: "Carbopol 940" (Lubrizol Corp.) | | | | | | |

As shown in Table 11, emollient creams using the vaseline-like composition of the present invention were clearly determined to have little stickiness and superior skin blendability while having moisture occlusion and skin spreadability comparable to vaseline.

### Lip Care Stick (Examples 33 to 36, Comparative Example 37)

Each component was weighed out according to the incorporated amounts shown in Table 12, and after warming and dissolving at 110°C and stirring to uniformity, the mixtures were poured into a metal mold and cooled to obtain lip care sticks.

### [Evaluation of Moisture Occlusion]

Moisture occlusion was evaluated using the same method as Examples 1 to 28 and Comparative Examples 1 to 35.

### [Sensory Evaluations]

Ten panelists were made to apply each sample to the lips to evaluate skin spreadability, stickiness and skin blendability. Favorable results were assigned a score of 2, average results were assigned a score of 1, and poor results were assigned a score of 0, and the parameters were evaluated as being acceptable when the total scores of the 10 panelists were evaluated as ⊚ or ○ as indicated below.
⊚: Total score of 18 or higher
○: Total score of 14 to 17
Δ: Total score of 10 to 13
×: Total score of 6 to 9
××: Total score of 5 or lower

**[Table 12]**

| Lip Care Stick Formulation and Evaluation Results (wt%) | | | | | | |
|---|---|---|---|---|---|---|
| Table 12 | | | | | | |
| Components | | Ex. 33 | Ex. 34 | Ex. 35 | Ex. 36 | Comp. Ex. 37 |
| Vaseline-like composition of Example 7 | | 50.0 | - | - | - | - |
| Vaseline-like composition of Example 12 | | - | 50.0 | - | - | - |
| Vaseline-like composition of Example 21 | | - | - | 50.0 | - | - |
| Vaseline-like composition of Example 26 | | - | - | - | 50.0 | - |
| Vaseline^{*1} | | - | - | - | - | 50.0 |
| Neopentyl glycol dicaprate | | 10.0 | 10.0 | 10.0 | 10.0 | 10.0 |
| Dipentaerythrityl pentaisostearate^{*2} | | 18.0 | 18.0 | 18.0 | 18.0 | 18.0 |
| Dipentaerythrityl hexa(hydroxystearate/ stearate/rosinate)^{*3} | | 10.0 | 10.0 | 10.0 | 10.0 | 10.0 |
| Candelilla wax | | 12.0 | 12.0 | 12.0 | 12.0 | 12.0 |
| Evaluation | Moisture occlusion | ⊚ | ⊚ | ⊚ | ⊚ | ⊚ |
| | Spreadability | ⊚ | ⊚ | ⊚ | ⊚ | ⊚ |
| | Stickiness | ⊚ | ○ | ⊚ | ○ | ×× |
| | Blendability | ⊚ | ⊚ | ⊚ | ⊚ | ×× |

| | | | | | | |
|---|---|---|---|---|---|---|
| *1: "Nomcoat W" (Nisshin OilliO Group, Ltd.) *2: "Saracos DP-518N" (Nisshin OilliO Group, Ltd.) *3: "Cosmol 168ARV" (Nisshin OilliO Group, Ltd.) | | | | | | |

As shown in Table 12, lip care sticks using the vaseline-like composition of the present invention were clearly determined to have little stickiness and superior skin blendability while having moisture occlusion and skin spreadability comparable to vaseline.

### W/O Emulsified Liquid Foundation (Example 37)

Components (A) were warmed and dissolved at 80°C in the incorporated amounts shown in Table 13, and the components (B) also warmed and dissolved at 80°C were slowly added thereto while stirring at 2000 rpm with a disperser. The mixture was then cooling to room temperature and degassed to obtain a W/O emulsified liquid foundation. The resulting W/O emulsified liquid foundation demonstrated superior moisture occlusion, superior skin spreadability, little stickiness and superior skin blendability.

**[Table 13]**

| W/O Emulsified Liquid Foundation | | (wt%) |
|---|---|---|
| Table 13 | Components | Ex. 37 |
| (A) | Vaseline-like composition of Example 8 | 7.0 |
| | Neopentyl glycol dicaprate | 3.0 |
| | 2-ethylhexyl para-methoxycinnamate | 2.0 |
| | Isostearic acid | 1.0 |
| | Dimethicone (10 cs) | 6.0 |
| | Cyclopentasiloxane | 6.0 |
| | Cetyl dimethicone copolyol^{*1} | 1.0 |
| | PEG-10 dimethicone^{*2} | 1.0 |
| | Talc | 2.7 |
| | Titanium oxide | 8.4 |
| | Iron oxide (yellow) | 0.6 |
| | Iron oxide (red) | 0.18 |
| | Iron oxide (black) | 0.12 |
| (B) | BG | 10.0 |
| | Glycerin | 2.0 |
| | Methyl parahydroxybenzoate | 0.2 |
| | Sodium chloride | 1.0 |
| | Sodium hyaluronate (1% aqueous solution) | 1.0 |
| | Ion exchange water | 46.8 |
| Total | | 100.0 |

| | | |
|---|---|---|
| *1: "Abil EM-90" (Evonik Industries, Ltd.) *2: "KF-6017" (Shin-Etsu Chemical Co., Ltd.) | | |

### Lipstick (Example 38)

All components were warmed and dissolved at 90°C in the incorporated amounts shown in Table 14, and after mixing to uniformity with a three-roller mixer, the mixture was poured into a metal mold and solidified by placing in a constant temperature bath at 0°C for 20 minutes to obtain a lipstick. The resulting lipstick demonstrated superior moisture occlusion, superior skin spreadability, little stickiness and superior skin blendability.

**[Table 14]**

| Table 14 | |
|---|---|
| Components | Example 38 |
| Vaseline-like composition of Example 1 | 50.0 |
| Dipentaerythrityl pentaisostearate^{*1} | 10.0 |
| Neopentyl glycol dicaprate | 13.0 |
| Dipentaerythrityl hexa(hydroxystearate/ stearate/rosinate) ^{*2} | 10.0 |
| Candelilla wax | 7.0 |
| Carnauba wax | 5.0 |
| Titanium oxide | 3.0 |
| Red dye no. 201 | 1.0 |
| Red dye no. 202 | 0.5 |
| Mica | 0.5 |
| Total | 100.0 |

| | |
|---|---|
| *1: "Saracos DP-518N" (Nisshin OilliO Group, Ltd.) *2: "Cosmol 168ARV" (Nisshin OilliO Group, Ltd.) | |

### Example 40 (Makeup Base)

A makeup base shown in Table 16 was prepared in accordance with the production method described below.

### (Production Method)

Components (A) were warmed to 70°C followed by slowly adding components (B) that were dissolved by warming to 70°C while dispersing by stirring at 2000 rpm with a desktop disperser mixer. The mixture was then cooled to 30°C while stirring.

The resulting makeup base demonstrated superior moisture occlusion, superior skin spreadability, little stickiness and superior skin blendability.

**[Table 16]**

| Makeup Base | | (wt%) |
|---|---|---|
| Table 16 | Components | Ex.40 |
| (A) | Vaseline-like composition of Example 11 | 10.0 |
| | Isononyl isononanoate | 2.0 |
| | Cyclopentasiloxane | 20.0 |
| | PEG-10 dimethicone^{*1} | 4.0 |
| | Titanium oxide | 3.0 |
| | Iron oxide | 0.2 |
| | Glyceryl tri(behenate/isostearate/eicosadioate)^{*2} | 4.0 |
| | Tocopherol | 0.05 |
| (B) | Ethanol | 5.0 |
| | 1,3-butylene glycol | 3.0 |
| | Sodium chloride | 0.5 |
| | Methyl parahydroxybenzoate | 0.2 |
| | Phenoxyethanol | 0.5 |
| | Ion exchange water | 47.55 |
| Total | | 100.0 |

| | | |
|---|---|---|
| *1: "KF-6017" (Shin-Etsu Chemical Co., Ltd.) *2: "Nomcoat SG" (Nisshin OilliO Group, Ltd.) | | |

### INDUSTRIAL APPLICABILITY

According to the present invention, a vaseline-like composition, which realizes moisture occlusion comparable to that of vaseline, has superior skin spreadability, superior skin blendability and little stickiness, and a cosmetic that incorporates this vaseline-like composition, can be provided.

## Claims

1. A vaseline-like composition, consisting of: the following components (C) or (E) at 5% by weight to 20% by weight, the following component (A) at 40% by weight to 90% by weight, and the following component (B) at 5% by weight to 50% by weight:
(A) one or more types of ester compounds selected from diisostearyl malate and diglyceryl triisostearate;
(B) a hydrocarbon wax having a melting point of 70°C to 130°C;
(C) a poly(12-hydroxystearic acid) having an average degree of polymerization of 3 to 20; and,
(E) a dimer dilinoleic acid derivative,
wherein the dimer dilinoleic acid derivative is an ester of an alcohol and one or more types of acids selected from dimer dilinoleic acid and hydrogenated dimer dilinoleic acid, and the alcohol is one or more types of alcohols selected from cholesterol, phytosterol and monovalent alcohols having 4 to 22 carbon atoms.

2. The vaseline-like composition according to claim 1, wherein the component (B) is one or more types of hydrocarbon wax selected from polyethylene wax, paraffin wax, ceresin wax and ozocerite.

3. The vaseline-like composition according to claim 1 or 2, wherein the average degree of polymerization of the poly(12-hydroxystearic acid) of the component (C) is 5 to 20.

4. The vaseline-like composition according to claim 3, wherein the alcohol contains one or more types of alcohols selected from cholesterol and phytosterol.

5. Use of the vaseline-like composition according to any of claims 1 to 4 in the manufacture of a cosmetic.

## Patentansprüche

1. Eine vaselinartige Zusammensetzung, bestehend aus den folgenden Komponenten (C) oder (E) in einem Anteil zwischen 5 Gewichts-% und 20 Gewichts-%, der folgenden Komponente (A) in einem Anteil zwischen 40 Gewichts-% bis 90 Gewichts-% und der folgenden Komponente (B) in einem Anteil zwischen 5 Gewichts-% bis 50 Gewichts-%:
(A) einer oder mehrere Typen von Esterverbindungen, die aus Diisostearyl-Malat und Diglyceryl-Triisostearat ausgewählt werden;
(B) ein Kohlenwasserstoffwachs mit einem Schmelzpunkt zwischen 70°C und 130°C;
(C) eine Poly(12-hydroxystearinsäure) mit einem durchschnittlichen Polymerisationsgrad zwischen 3 und 20; und,
(E) ein Dimer-Dilinolsäure-Derivat, wobei es sich bei dem Dimer-Dilinolsäure-Derivat um einen Alkoholester und einen oder mehrere Säuretypen handelt, die aus Dimer-Dilinolsäure und hydrierter Dimer-Dilinolsäure ausgewählt werden, und wobei es sich beim Alkohol um einen oder mehrere Alkoholtypen handelt, die aus Cholesterin, Phytosterin und monovalenten Alkoholen mit zwischen 4 und 22 Kohlenstoffatomen ausgewählt werden.

2. Vaselinartige Zusammensetzung nach Anspruch 1, wobei es sich bei der Komponente (B) um einen oder mehrere Typen von Kohlenstoffwachs handelt, ausgewählt aus Polyethylenwachs, Paraffinwachs, Ceresin und Ozokerit.

3. Vaselinartige Zusammensetzung nach Anspruch 1 oder 2, wobei der durchschnittliche Polymerisationsgrad der Poly(12-hydroxystearinsäure) der Komponente (C) zwischen 5 und 20 liegt.

4. Vaselinartige Zusammensetzung nach Anspruch 3, wobei der Alkohol einen oder mehrere Typen von Alkohol enthält, die aus Cholesterin und Phytosterin ausgewählt werden.

5. Einsatz der vaselinartigen Zusammensetzung nach einem der Ansprüche 1 bis 4 bei der Herstellung eines Kosmetikpräparats.

## Revendications

1. Composition de type vaseline, qui contient : les composants (C) ou (E) suivants à raison de 5 % en poids à 20 % en poids, le composant (A) suivant à raison de 40 % en poids à 90 % en poids, et le composant (B) suivant à raison de 5 % en poids à 50 % en poids :
(A) un ou plusieurs types de composés esters choisis parmi le malate de diisostéaryle et le triisostéarate de diglycéryle ;
(B) une cire hydrocarbonée ayant un point de fusion de 70°C à 130°C ;
(C) de l'acide poly(12-hydroxystéarique) ayant un degré moyen de polymérisation de 3 à 20 ; et,
(E) un dérivé d'acide dilinoléique dimère,
dans lequel le dérivé d'acide dilinoléique dimère est un ester d'un alcool et d'un ou plusieurs types d'acides choisis parmi l'acide dilinoléique dimère et l'acide dilinoléique dimère hydrogéné, et l'alcool est un ou plusieurs types d'alcools choisis parmi le cholestérol, le phytostérol et des alcools monovalents ayant 4 à 22 atomes de carbone.

2. Composition de type vaseline selon la revendication 1, dans laquelle le composant (B) est un ou plusieurs types de cire hydrocarbonée choisis parmi la cire de polyéthylène, la cire de paraffine, la cire de cérésine et l'ozocérite.

3. Composition de type vaseline selon la revendication 1 ou 2, dans laquelle le degré moyen de polymérisation de l'acide poly(12-hydroxystéarique) du composant (C) est de 5 à 20.

4. Composition de type vaseline selon la revendication 3, dans laquelle l'alcool contient un ou plusieurs types d'alcools choisis parmi le cholestérol et le phytostérol.

5. Utilisation de la composition de type vaseline selon l'une quelconque des revendications 1 à 4 dans la fabrication d'un cosmétique.
